# EUROPEAN PATENT APPLICATION

(11) **EP 1 728 808 A1**
(43) Date of publication of application: **06.12.2006**
(21) Application number: 06010447.8
(22) Date of filing: 20.05.2006
(51) Int. Cl.: C08G 18/80, C09D 175/04, C07C 265/16

(54) **Polyisocyanates blocked with diisopropyl malonate and their use in one-component coating compositions**

(30) Priority: 03.06.2005 JP 2005164365
(71) Applicant: Bayer MaterialScience AG, 51368 Leverkusen (DE)
(72) Inventor: Katamura, Kouchi, Kobe-City Hyogo 658-0898 (JP); Iwanaka, Toshihiro, Amagasaki-City Hyogo 661-0003 (JP); Shigemori, Tomokazu, Neyagawa-City Osaka-Fu 572-0804 (JP); Tabana, Hisafumi, Suita-City Osaka-Fu 565-0875 (JP)

(57) **Abstract**

The present invention relates to a blocked polyisocyanate composition containing
A) a blocked polyisocyanate which is the reaction product of
i) a polyisocyanate prepared from an aliphatic and/or alicyclic diisocyanate and
ii) a malonic acid diester blocking agent containing at least 90 equivalent %, based on the total equivalents of blocking agent, of diisopropyl malonate, and

B) a mono-functional active hydrogen-containing compound.

The present invention also relates to a one-component coating composition containing these blocked polyisocyanate compositions and a polyhydroxyl compound.

## Description

### BACKGROUND OF THE INVENTION

The present invention relates to polyisocyanates blocked with diisopropyl malonate and the use of these blocked polyisocyanates in one-component coating compositions.

### SUMMARY OF THE INVENTION

The present invention relates to a blocked polyisocyanate composition containing
A) a blocked polyisocyanate which is the reaction product of
   i) a polyisocyanate prepared from an aliphatic and/or alicyclic diisocyanate and
   ii) a malonic acid diester blocking agent containing at least 90 equivalent %, based on the total equivalents of blocking agent, of diisopropyl malonate, and
B) a mono-functional active hydrogen-containing compound.

The present invention also relates to a one-component coating composition containing these blocked polyisocyanate compositions and a polyhydroxyl compound.

### DETAILED DESCRIPTION OF THE INVENTION

The blocked polyisocyanate compositions according to the invention possess improved resistance to crystallization and good storage stability, particularly at low temperatures. In addition, they can be mixed with polyhydroxyl compounds to form one-component coating compositions which also possess improved resistance to crystallization and good storage stability, particularly at low temperatures.

Suitable polyisocyanates i) for preparing the blocked polyisocyanates are polyisocyanates which contain isocyanurate, uretdione, biuret, urethane, allophanate and/or iminooxadiazine dione groups, and are prepared from aliphatic and/or alicyclic diisocyanates. These polyisocyanates and methods for their preparation are well known in the art of polyurethane coatings.

Suitable aliphatic and/or alicyclic diisocyanates for preparing the preceding polyisocyanates are also well known in the art of polyurethane coatings and include 1,6-hexamethylene diisocyanate, 1-isocyanato-3-isocyanatomethyl-3,5,5-trimethyl-cyclohexane (isophorone diisocyanate or IPDI), bis-(4-isocyanatocyclohexyl)-methane, 1-isocyanato-1-methyl-4(3)-isocyanatomethyl cyclohexane, 2,4- and/or 2,6-hexahydrotoluylene diisocyanate, and mixtures thereof. Preferred aliphatic and/or alicyclic diisocyanates are 1,6-hexamethylene diisocyanate, 1-isocyanato-3-isocyanatomethyl-3,5,5-trimethyl-cyclohexane (isophorone diisocyanate or IPDI), bis-(4-isocyanatocyclohexyl)-methane and mixtures thereof more preferably 1,6-hexamethlene diisocyanate.

At least 90 equivalent % of the blocking agent, based on the total equivalents of blocking agent, is diisopropyl malonate. The remainder of the blocking agent is selected from malonic acid diesters, preferably malonic acid dialkylesters, especially those having 1 to 8, more preferably 1 to 4 carbon atoms in each alkyl group. An especially preferred malonic acid diester other than diisopropyl malonate is diethyl malonate.

The blocked polyisocyanate compositions also contain a mono-functional active hydrogen-containing compound, preferably a monoalcohol, especially those having 1 to 12, preferably 1 to 8 carbon atoms. Examples include ethanol and the isomeric propanols, butanols, hexanols and octanols. Especially preferred is isobutanol.

In addition to the preceding components the blocked polyisocyanate compositions may also contain the known organic solvents from polyurethane chemistry with the exception of aromatic hydrocarbons.

In addition to the blocked polyisocyanate compositions, the one-component coating compositions according to the invention also contain a polyhydroxyl compound. Examples include polyhydroxy polyesters, polyhydroxy polyethers, polyhydroxy polyacrylates, polyhydroxy polylactones, polyhydroxy polyurethanes, polyhydroxy polyepoxides and optionally low molecular weight, polyhydric alcohols known from polyurethane coatings technology.

To prepare the coating compositions the amount of the blocked polyisocyanate composition and the polyhydroxyl compound are selected to provide equivalent ratios of isocyanate groups in blocked form to hydroxyl groups of about 0.8 to 3, preferably about 0.9 to 1.5. The one-component coating compositions are cured at elevated temperatures.

The coating compositions may also contain other additives such as pigments, dyes, fillers, levelling agents and solvents. The coating compositions may be applied to the substrate to be coated in solution or from the melt by conventional methods such as painting, rolling, pouring or spraying.

The invention is further illustrated, but is not intended to be limited by the following examples in which all parts and percentages are by weight unless otherwise specified.

### EXAMPLES

The following evaluation methods were used in the examples.

### The evaluation for depression of crystallization of a blocked polyisocyanate composition (storage stability at low temperatures)

500g of the blocked polyisocyanate composition were stored in a 500 ml metal can for 7days at -10 degree C, and then the solution state of the composition was observed and determined as follows:
- o (good):: no deposition of a crystallized substance or a gelled compound was observed.
- x (bad):: deposition of a crystallized substance or a gelled compound was observed.

### The cure property of one-component coating composition

### (gel fraction)

The cured paint film was baked for 30 min. in a drying oven kept at 90°C and then dipped in acetone solution for 24hr. Its weight was measured and residual weight rate (%) was calculated and determined as shown below;
- o (good):: residual weight rate (%) was more than 90% meaning a high cure property.
- x (bad):: residual weight rate (%) was less than 90% meaning a low cure property.

### The storage stability of a one-component coating composition

500g of a blocked polyisocyanate composition were stored in a 500ml metal can for 10 days at 50°C and the change of viscosity was measured by Ford cup #4 at 25°C. The storage stability of the composition was determined as shown below:
- o (good):: The viscosity was less than 1.5 times the initial viscosity, which was acceptable.
- x (bad):: The viscosity was more than 1.5 times the initial viscosity, which was not acceptable.
Remarks: pbw = part by weight

### Example 1

100 pbw of polyisocyanate I containing isocyanurate groups and prepared from 1,6-hexamethylene diisocyanate (Sumidur N3300, manufactured by Sumika Bayer Urethane Co.,Ltd., % NCO = 21.8%, viscosity = 3,000 mPa.s/25°C, average functionality = 3.5), 98 pbw of diisopropyl malonate (100 equivalent %, based on isocyanate groups), 50 pbw of n-butyl acetate, 0.7 pbw of 28% sodium metylate (=sodium methoxide) solution (0.196 pbw as sodium metylate) were added into a four-neck flask equipped with agitator, thermometer and reflux condenser at room temperature under an atmosphere of nitrogen and reacted for 8 hrs. at 70°C. Then 82 pbw of isobutanol were added and continuously reacted for 2 hrs. at 50°C. The properties of the resulting blocked polyisocyanate composition were as follows:

| | |
|---|---|
| NCO content | 6.6% |
| Viscosity | 130 mPa.s/25°C |
| Resin solids | 60% |

The evaluation for depression of crystallization of the blocked polyisocyanate composition is shown in Table 1. Because no deposition of a crystallized substance or a gelled compound was observed by the evaluation for depression of crystallization at -10°C for more than 7 days, the evaluation result was good.

A one-component composition was prepared by mixing the preceding blocked polyisocyanate composition and an acrylic polyol (Desmophen A665, manufactured by Bayer MaterialScience, hydroxyl number =106 mgKOH/g, resin content = 70%) at an equivalent ratio of NCO groups of the blocked polyisocyanate to hydroxyl groups of the polyol of 1:1, and then a mixture of butyl acetate and propylene glycol monomethyl ether acetate (1:1 by weight) was added. The resulting one-component coating composition had a total resin solids content of 43%. The one-component coating composition was coated at a dry film thickness of 50 µm and then the coating was baked for 30 min. in drying oven kept at 80 - 90°C. The cure property (gel fraction) of cured film and the storage stability of the one-component coating composition were evaluated. The results of the cure property (gel fraction) and the storage stability were good as shown in Table 1.

### Example 2

A blocked polyisocyanate composition was prepared as set forth in Example 1 except that the composition was prepared using 88 pbw of diisopropyl malonate (90 equivalent %, based on isocyanate groups), 8 pbw of diethyl malonate (10 equivalent %, based on isocyanate groups), 49 pbw of n-butyl acetate and 81 pbw of isobutanol. The properties of the resulting blocked polyisocyanate composition were as follows:

| | |
|---|---|
| NCO content | 6.7% |
| Viscosity | 130 mPa.s/25°C |
| Resin solids | 60% |

The evaluation for depression of crystallization of the blocked polyisocyanate composition is shown in Table 1. Because no deposition of a crystallized substance or a gelled compound was observed by the evaluation for depression of crystallization at -10°C for more than 7 days, the evaluation result was good.

A one-component coating composition having a resin solids content of 43% was prepared from the preceding blocked polyisocyanate composition using the same procedure as in Example 1. The evaluation of the cure property (gel fraction) and the storage stability were carried out using the same procedure as in Example 1. The results were good as shown in Table 1.

### Comparative Example 1

The blocked polyisocyanate composition was prepared by the same preparation method as Example 1 except that the composition was changed to 78 pbw of diisopropyl malonate (80 equivalent %, based on isocyanate groups), 17 pbw of diethyl malonate (20 equivalent %, based on isocyanate groups), 49 pbw of n-butyl acetate and 80 pbw of isobutanol. The properties of the resulting blocked polyisocyanate composition were as follows:

| | |
|---|---|
| NCO content | 6.7% |
| Viscosity | 140 mPa.s/25°C |
| Resin solids | 60% |

The evaluation for depression of crystallization of the blocked polyisocyanate composition is shown in Table 1. Because deposits of a crystallized substance and a gelled compound were observed by the evaluation for depression of crystallization at -10°C for more than 7 days, the evaluation result was unsatisfactory.

A one-component coating composition having a resin solids content of 43% was prepared from the preceding blocked polyisocyanate composition using the same procedure as in Example 1. The evaluation of the cure property (gel fraction) and the storage stability were carried out using the same procedure as in Example 1. Although the result of the storage stability was good, the result of the gel fraction was unsatisfactory as shown in Table 1.

### Comparative Example 2

The blocked polyisocyanate composition was prepared by the same preparation method as Example 1 except that the composition was changed to 83 pbw of diethyl malonate (100 equivalent %, based on isocyanate groups), 46 pbw of n-butyl acetate and 76 pbw of isobutanol. The properties of the resulting blocked polyisocyanate composition were as follows:

| | |
|---|---|
| NCO content | 7.1% |
| Viscosity | 140 mPa.s/25°C |
| Resin solids | 60% |

The evaluation for depression of crystallization of the blocked polyisocyanate composition is shown in Table 1. Because deposits of a crystallized substance and a gelled compound were observed by the evaluation for depression of crystallization at -10°C for more than 7 days, the evaluation result was unsatisfactory.

A one-component coating composition having a resin solids content of 43% was prepared from the preceding blocked polyisocyanate composition using the same procedure as in Example 1. The evaluation of the cure property (gel fraction) and the storage stability were carried out using the same procedure as in Example 1. Although the result of the storage stability was good, the result of the gel fraction was unsatisfactory as shown in Table 1.

### Comparative Example 3

The blocked polyisocyanate composition was prepared by the same preparation method as Example 1 except that the composition was changed to 132 pbw of n-butyl acetate and no isobutanol was used. The properties of the prepared blocked polyisocyanate composition were as follows:

| | |
|---|---|
| NCO content | 6.6% |
| Viscosity | 90 mPa.s/25°C |
| Resin solids | 60% |

The evaluation for depression of crystallization of the blocked polyisocyanate composition is shown in Table 1. Because deposits of a crystallized substance and a gelled compound were observed by the evaluation for depression of crystallization at -10°C for more than 7 days, the evaluation result was unsatisfactory.

A one-component coating composition having a resin solids content of 43% was prepared from the preceding blocked polyisocyanate composition using the same procedure as in Example 1. The evaluation of the cure property (gel fraction) and the storage stability were carried out using the same procedure as in Example 1. Although the result of the gel fraction was good, the result of the storage stability was unsatisfactory as shown in Table 1.

### Comparative Example 4

The blocked polyisocyanate composition was prepared by the same preparation method as Example 1 except that the composition was changed to 64 pbw of diisopropyl malonate (65 equivalent %, based on isocyanate groups), 24 pbw of ethyl acetoacetate (35 equivalent %, based on isocyanate groups), 47 pbw of n-butyl acetate and 78 pbw of isobutanol. The properties of the resulting blocked polyisocyanate composition were as follows:

| | |
|---|---|
| NCO content | 6.8% |
| Viscosity | 110 mPa.s/25°C |
| Resin solids | 60% |

The evaluation for depression of crystallization of the blocked polyisocyanate composition is shown in Table 1. Because deposits of a crystallized substance and a gelled compound were observed by the evaluation for depression of crystallization at -10°C for more than 7 days, the evaluation result was unsatisfactory.

A one-component coating composition having a resin solids content of 43% was prepared from the preceding blocked polyisocyanate composition using the same procedure as in Example 1. The evaluation of the cure property (gel fraction) and the storage stability were carried out using the same procedure as in Example 1. Although the result of the storage stability was good, the result of the gel fraction was unsatisfactory as shown in Table 1.

**TABLE 1**

| | Example | | Comparative Example | | | |
|---|---|---|---|---|---|---|
| | 1 | 2 | 1 | 2 | 3 | 4 |
| Polyisocyanate (pbw) | 100 | 100 | 100 | 100 | 100 | 100 |
| blocking agent (pbw) | | | | | | |
| diisopropyl malonate | 98 | 88 | 78 | | 98 | |
| diethyl malonate | | 8 | 17 | 83 | | 64 |
| ethyl acetoacetic | | | | | | 24 |
| equivalent (%) to isocyanate | | | | | | |
| group of polyisocyanate | | | | | | |
| diisopropyl malonate | 100 | 90 | 80 | | 100 | |
| diethyl malonate | | 10 | 20 | 100 | | 65 |
| ethyl acetoacetic | | | | | | 35 |
| catalyst (pbw) | | | | | | |
| 28% sodium metylate | 7 | 7 | 7 | 7 | 7 | 7 |
| Solvent component (pbw) | | | | | | |
| n-butyl acetate | 50 | 49 | 49 | 46 | 132 | 47 |
| monofunctional active hydrogen- | | | | | | |
| containing compound (pbw) | | | | | | |
| isobutanol | 82 | 81 | 81 | 76 | | 78 |
| blocked polyisocyanate | | | | | | |
| composition | | | | | | |
| evaluation of for depression of | o | o | x | x | x | x |
| crystallization (-10 degree C X | | | | | | |
| above 7 days) | | | | | | |
| One-component coating | | | | | | |
| composition | | | | | | |
| (1) cure property (gel fraction) | o | o | x | x | o | x |
| 90 degree C | 92 | 91 | 89 | 88 | 92 | 80 |
| (2) storage stability | | | | | | |
| (50 degree C x 10 days) | o | o | o | o | x | o |

Although the invention has been described in detail in the foregoing for the purpose of illustration, it is to be understood that such detail is solely for that purpose and that variations can be made therein by those skilled in the art without departing from the spirit and scope of the invention except as it may be limited by the claims.

## Claims

1. A blocked polyisocyanate composition comprising
A) a blocked polyisocyanate which is the reaction product of
i) a polyisocyanate prepared from an aliphatic and/or alicyclic diisocyanate and
ii) a malonic acid diester blocking agent containing at least 90 equivalent %, based on the total equivalents of blocking agent, of diisopropyl malonate, and
B) a mono-functional active hydrogen-containing compound.

2. The blocked polyisocyanate composition of Claim 1, wherein the composition additionally contains an organic solvent other than an aromatic hydrocarbon.

3. The blocked polyisocyanate composition of Claim 1, wherein component i) comprises a polyisocyanate containing isocyanurate groups that is prepared from an aliphatic diisocyanate.

4. The blocked polyisocyanate composition of Claim 1, wherein component B) comprises a monoalcohol.

5. The blocked polyisocyanate composition of Claim 3, wherein component B) comprises a monoalcohol.

6. A one-component coating composition comprising a blocked polyisocyanate composition and a polyhydroxyl compound, wherein the blocked polyisocyanate composition comprises
A) a blocked polyisocyanate which is the reaction product of
i) a polyisocyanate prepared from an aliphatic and/or alicyclic diisocyanate and
ii) a malonic acid diester blocking agent containing at least 90 equivalent %, based on the total equivalents of blocking agent, of diisopropyl malonate, and
B) a mono-functional active hydrogen-containing compound.

7. The one-component coating composition of Claim 6, wherein the composition additionally contains an organic solvent other than an aromatic hydrocarbon.

8. The one-component coating composition of Claim 6, wherein component i) comprises a polyisocyanate containing isocyanurate groups that is prepared from an aliphatic diisocyanate.

9. The one-component coating composition of Claim 6, wherein component B) comprises a monoalcohol.

10. The one-component coating composition of Claim 8, wherein component B) comprises a monoalcohol.
